(19) Europäisches Patentamt
European Patent Office
Office européen des brevets

(11) Veröffentlichungsnummer: **0 395 581 A1**

(12) # EUROPÄISCHE PATENTANMELDUNG

(21) Anmeldenummer: **90810306.2**

(22) Anmeldetag: **18.04.90**

(51) Int. Cl.⁵: **C07C 381/08, C07C 313/24, C07C 381/00, A01N 41/00**

(30) Priorität: **27.04.89 CH 1598/89**

(43) Veröffentlichungstag der Anmeldung: **31.10.90 Patentblatt 90/44**

(84) Benannte Vertragsstaaten: **BE CH DE DK ES FR GB IT LI NL**

(71) Anmelder: **CIBA-GEIGY AG**
**Klybeckstrasse 141**
**CH-4002 Basel(CH)**

(72) Erfinder: **Drabek, Jozef, Dr.**
**Benkenstrasse 12**
**CH-4104 Oberwil(CH)**

(54) **N-Sulfenyl- und N-sulfinyl-N,N'-diacylhydrazide.**

(57) N-Sulfenyl- und N-Sulfinyl-N,N′-diacylhydrazide der Formel I

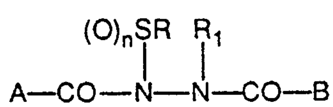

(I)

worin

A und B unabhängig voneinander Phenyl oder Naphthyl, welches unsubstituiert oder ein-oder mehrfach durch Halogen, $C_1$-$C_4$ Alkyl, $C_1$-$C_4$ Alkoxy, $C_1$-$C_4$ Haloalkyl, $C_1$-$C_4$ Haloalkoxy, $C_1$-$C_4$ Alkyl-$S(O)_n$-, $C_1$-$C_4$ Haloalkyl-$S(O)_n$-, Nitro, Cyan, Hydroxyl, Carboxyl oder $C_1$-$C_4$ Alkylcarbonyl oder $C_1$-$C_4$ Alkoxycarbonyl substituiert sind,

n Null oder 1,

R $C_1$-$C_6$ Alkyl, Phenyl oder Naphthyl die unsubstituiert oder wie oben unter A und B angegeben substituiert sein können, oder einen Rest $-N(R_2)_2$, $-N(R_2)COR_2$, $-N(R_2)COOR_2$, $-N(R_2)SO_2R_2$ oder $-(S)_n-N(COA)N(R_1)-COB$,

$R_1$ $C_1$-$C_6$ Alkyl, $C_1$-$C_6$ Haloalkyl, $C_3$-$C_6$ Alkenyl, $C_3$-$C_6$ Haloalkenyl, $C_3$-$C_6$ Alkynyl, $C_3$-$C_7$ Cycloalkyl, Phenyl oder Naphthyl, welche unsubstituiert oder wie oben unter A und B angegeben substituiert sind und

$R_2$ $C_1$-$C_6$ Alkyl, $C_1$-$C_6$ Haloalkyl, $C_2$-$C_6$ Alkoxyalkyl, $C_3$-$C_6$ Alkenyl, Phenyl oder Naphthyl, welche unsubstituiert oder wie oben unter A und B angegeben substituiert sind,

bedeuten, haben gute insektizide und akarizide Wirkung. Verfahren zu ihrer Herstellung sowie ihre Verwendung zur Schädlingsbekämpfung in der Landwirtschaft werden beschrieben.

EP 0 395 581 A1

## N-Sulfenyl- und N-Sulfinyl-N,N′-diacylhydrazide

Die vorliegende Erfindung betrifft neue N-Sulfenyl- und N-Sulfinyl-N,N′-diacylhydrazide, Verfahren zu ihrer Herstellung und Mittel und Verfahren zu ihrer Verwendung in der Schädlingsbekämpfung.

N,N′-Dibenzoyl- und N,N′-Dinaphthoylhydrazide, die keine Schwefelatome enthalten und insektizide Wirkung besitzen, sind bereits aus EP-A-228,564, EP-A-236,618 und EP-A-245 ,950 bekannt.

Demgegenüber unterscheiden sich die erfindungsgemässen N-Sulfenyl- und N-Sulfinyl-diacylhydrazide strukturell durch einen über Schwefel oder Sulfinyl gebundenen Alkyl-, Aryl- oder Aminorest und durch eine breite insektizide, akarizide und ovizide Wirkung, die derjenigen ähnlicher bekannter Hydrazide in Bezug auf Wirkungsdauer, Wirkungsstärke und Wirkungsbreite überlegen ist.

Die erfindungsgemässen N-Sulfenyl- und N-Sulfinyl-N,N′-diacylhydrazide entsprechen der Formel I

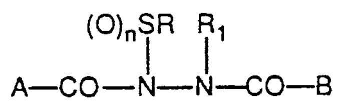

(I)

worin

A und B unabhängig voneinander Phenyl oder Naphthyl, welches unsubstituiert oder ein-oder mehrfach durch Halogen, $C_1$-$C_4$ Alkyl, $C_1$-$C_4$ Alkoxy, $C_1$-$C_4$ Haloalkyl, $C_1$-$C_4$ Haloalkoxy, $C_1$-$C_4$ Alkyl-$S(O)_n$-, $C_1$-$C_4$ Haloalkyl-$S(O)_n$-, Nitro, Cyan, Hydroxyl, Carboxyl oder $C_1$-$C_4$ Alkylcarbonyl oder $C_1$-$C_4$ Alkoxycarbonyl substituiert sind;

n Null oder 1;

R $C_1$-$C_6$ Alkyl, Phenyl oder Naphthyl die unsubstituiert oder wie oben unter A und B angegeben substituiert sein können, oder einen Rest $-N(R_2)_2$, $-N(R_2)COR_2$, $-N(R_2)COOR_2$, $-N(R_2)SO_2R_2$ oder $-(S)_n-N(COA)N(R_1)-$COB;

$R_1$ $C_1$-$C_6$ Alkyl, $C_1$-$C_6$ Haloalkyl, $C_3$-$C_6$ Alkenyl, $C_3$-$C_6$ Haloalkenyl, $C_3$-$C_6$ Alkynyl, $C_3$-$C_7$ Cycloalkyl, Phenyl oder Naphthyl, welche unsubstituiert oder wie oben unter A und B angegeben substituiert sind und

$R_2$ $C_1$-$C_6$ Alkyl, $C_1$-$C_6$ Haloalkyl, $C_2$-$C_6$ Alkoxyalkyl, $C_3$-$C_6$ Alkenyl, Phenyl oder Naphthyl, welche unsubstituiert oder wie oben unter A und B angegeben substituiert sind,

bedeuten.

Besonders gute insektizide Wirkung zeigten die Verbindungen der Formel Ia

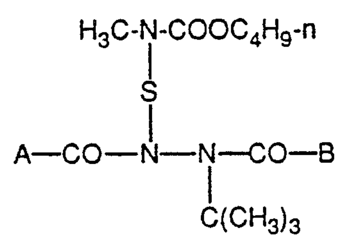

(Ia),

worin A und B unabhängig voneinander einen Phenyl- oder Naphthylrest, welcher unsubstituiert oder, wie oben angegeben, substituiert ist, bedeuten, insbesondere auch die Verbindungen N,N′-Dibenzoyl-N-[(N″-methyl-N″-n-butoxycarbonyl)-aminosulfenyl]-N′-tert.butyl-hydrazid und N,N′-Dibenzoyl-N-(2″-cyano-prop-2″-yl-dithio)-N′-tert.butyl-hydrazid.

Bevorzugt sind erfindungsgemäss Verbindungen der Formel I, worin n für Null steht.

Vorzugsweise sind substituierte Phenyl- oder Naphthylreste unter A, B, R, $R_1$ und $R_2$ ein-oder zweifach substituiert.

Hervorzuheben sind weiterhin diejenigen erfindungsgemässen Verbindungen der Formel I, die dadurch gekennzeichnet sind, dass A und B unabhängig voneinander Phenyl oder mit 1 bis 2 Halogenatomen substituiertes Phenyl, oder dadurch gekennzeichnet sind, dass R $C_1$-$C_4$-Alkyl oder einen Rest $N(R_2)_2$ oder $N(R_2)COOR_2$ bedeutet, wobei $R_2$ für $C_1$-$C_4$-Alkyl steht, oder dadurch gekennzeichnet sind, dass $R_1$ $-C(CH_3)_3$ bedeutet.

Die Erfindung umfasst auch tautomere Formen und Gemische von Tautomeren in denen diese Verbindungen auftreten können.

Unter dem Begriff Alkyl allein oder als Bestandteil eines komplexen Substituenten sind im Rahmen der vorliegenden Erfindung geradkettige und verzweigte Alkylreste und je nach Zahl der angegebenen Kohlenstoffatome beispielsweise folgende Gruppen zu verstehen: Methyl, Aethyl, Propyl, Butyl, Pentyl usw. sowie ihre Isomeren, wie z.B. Isopropyl, Isobutyl, tert.-Butyl, Isoamyl, tert. Pentyl usw..

Alkenylreste können geradkettig oder verzweigt sein und umfassen beispielsweise folgende Gruppen: Alkyl, Methalkyl, Butenyl, Butadienyl, 2-Methylbutenyl, 3-Methylbutenyl, Pentenyl, 2-Ethylalkyl, Hexenyl oder Hexadienyl usw., bevorzugt sind Allyl und Methallyl.

Alkynylreste können ebenfalls verzweigt oder geradkettig sein und umfassen beispielsweise die Gruppen Propynyl, Butynyl, Pentynyl, 1-Methylpropynyl, Hexinyl usw., bevorzugt is Propynyl.

Unter Halogen sind Fluor, Chlor, Brom und Jod, vorzugsweise Fluor oder Brom, zu verstehen.

Unter dem Begriff Haloalkyl im Rahmen der vorliegenden Erfindung sind geradkettige und verzweigte Alkylreste, wie Methyl, Aethyl, n-Propyl, Isopropyl und die isomeren Butylreste zu verstehen, die mit bis zu 9 unterschiedlichen oder gleichartigen Halogenatomen substituiert sind, wobei es sich um perhalogenierte Alkylreste oder auch um solche handeln kann, deren Wasserstoffatome nur teilweise durch Halogen substituiert sind. Als Halogenalkyle sind beispielsweise die Perfluoralkylradikale Trifluormethyl, Pentafluorethyl oder Heptafluor-n-propyl zu nennen.

Die erfindungsgemässen Verbindungen der Formel I können nach an sich bekannten Verfahren hergestellt werden, wie z.B. den folgenden:

Ein N,N′-Diacylhydrazid der Formel II

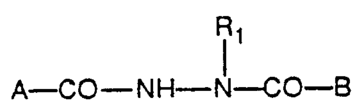 (II),

worin A, B und $R_1$ die unter der Formel I gegebenen Bedeutungen haben, wird vorzugsweise in einem inerten organischen Lösungsmittel und in Gegenwart der mindestens äquimolaren Menge einer Base, mit einem Halomerkaptan resp. einer Halosulfinylverbindung der Formel III

$R-[(O)_nS]_{\overline{m}}-Hal$ (III),

worin R die vorstehend unter der Formel I angegebenen Bedeutungen hat, Hal ein Halogenatom, vorzugsweise Chlor oder Brom, n Null oder 1, m 1 oder 2 bedeuten, umgesetzt.

Als Lösungsmittel kommen für diese Umsetzung z.B. Tetrahydrofuran, Aether, Aceton, Methyläthylketon, Dioxan, Aethylacetat, Toluol oder Pyridin in Betracht. Geeignete Basen sind z.B. Natriumhydrid oder organische Basen, wie Trimethylamin, Triäthylamin, Pyridin oder N-Methylpyridin. Die Umsetzung wird bei Temperaturen zwischen -20 bis +80 °C, vorzugsweise -10 bis +50 °C durchgeführt.

Die als Ausgangsstoffe für diese Umsetzung benötigten Diacylhydrazide der Formel II sind entweder bekannt oder können nach bekannten Methoden hergestellt werden (siehe z.B. Helv. Chim. Acta 61 (1968) p. 1977, EP-A-228,569 und EP-A-236,618). Die als Ausgangsstoffe benötigten Halomerkaptane oder Halosulfinyl-Verbindungen der Formel III sind ebenfalls bekannt oder können analog bekannten Verfahren hergestellt werden (siehe z.B. "Progress in Pesticide Biochemistry and Toxicology" Vol 5, p. 35-86, John Wiley and Sons, New York 1985).

Ein Weg zur Herstellung von denjenigen N,N′-Diacylhydraziden der Formel I, worin A und B die gleiche Bedeutung haben, besteht darin, dass man ein Hydrazin der Formel IV

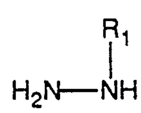 (IV),

worin $R_1$ die unter der Formel I angegebene Bedeutung hat, vorzugsweise in einem inerten organischen Lösungsmittel und in Gegenwart der mindestens äquimolaren Menge einer Base mit einem Halomerkaptan oder einer Halosulfinyl-Verbindung der Formel III kondensiert,

$R-[(O)_nS]_{\overline{m}}-Hal$ (III),

worin m, n und $R_1$ die oben angegebenen Bedeutungen haben, und das auf diese Weise er haltene Sulfenylhydrazid der Formel V

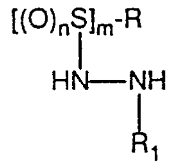

(V),

worin m, n, R und $R_1$ die oben angegebenen Bedeutungen haben, dann, vorzugsweise in einem inerten organischen Lösungsmittel und in Gegenwart der mindestens äquimolaren Menge einer Base mit der, zweifach molaren Menge eines Arylcarbonylhalides der Formel VI

A-CO-Hal (VI),

worin A die unter der Formel I angegebene Bedeutung hat und Hal ein Halogenatom, vorzugsweise Chlor oder Brom bedeutet, umsetzt.

Auch für diese Umsetzung eignen sich z.B. die Lösungsmittel Tetrahydrofuran, Aether, Aceton, Methyläthylketon, Dioxan, Aethylacetat, Toluol oder Pyridin. Als Basen werden vorzugsweise organische Basen, tertiäre Amine, Pyridin oder Methylpyridin verwendet. Die vorstehenden Reaktionen finden bei Temperaturen zwischen -20 bis +80° C, vorzugsweise -20° C bis +40° C, statt. Während der exothernen Anfangsphase wird vorteilhafterweise gekühlt und später zur Vervollständigung der Umsetzung kann leicht erwärmt werden.

Die als Ausgangsprodukt benötigten Hydrazine der Formel IV sind bekannt oder können nach bekannten Methoden hergestellt werden (siehe z.B. Houben-Weyl, Vol 10 sowie die schweizerische Patentschrift No. 309,770).

Ein weiterer Weg zur Herstellung der N-Sulfenyl- oder N-Sulfinyl-N,N′-diacylhydrazide der Formel I besteht darin, dass man ein Acylhydrazid der Formel VII

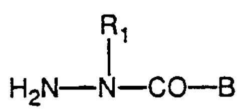

(VII),

worin B und $R_1$ die unter der Formel I angegebenen Bedeutungen haben, vorzugsweise in einem inerten organischen Lösungsmittel und in Gegenwart der mindestens äquimolaren Menge einer Base, zuerst mit einem Halomerkaptan oder einer Halosulfinylverbindung der Formel III kondensiert

$R-[(O)_nS]_m-Hal$ (III),

worin Hal, m, n und R die oben angegebenen Bedeutungen haben, und das erhaltene N-Sulfenyl- oder N-Sulfinyl-N′-acyl-hydrazid der Formel VIII

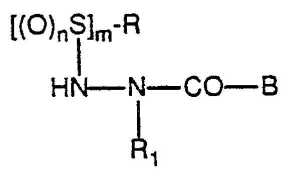

(VIII),

worin B, m, n, R und $R_1$ die oben angegebenen Bedeutungen haben, dann, vorzugsweise in einem inerten organischen Lösungsmittel und in Gegenwart der mindestens äquimolaren Menge einer Base, mit einem Arylcarbonylhalid der Formel VI

A-CO-Hal (VI),

worin A die unter der Formel I angegebene Bedeutung hat und Hal für ein Halogenatom, vorzugsweise Chlor oder Brom, steht, umsetzt.

Für diese Umsetzungen kommen die obenerwähnten Lösungsmittel, aber auch chlorierte Kohlenwasserstoffe, wie Chloroform, Methylenchlorid, Tetrachlorkohlenstoff in Betracht. Als Basen eignen sich neben Natriumhydrid, tertiäre Amine, Pyridin und N-Methylpyridin. Die Reaktionstemperaturen liegen zwischen -20° C und +30° C, vorteilhafterweise zwischen -10° C und +50° C. Die als Ausgangsprodukte der Formel VII benötigten Acylhydrazide sind bekannt oder können nach bekannten Methoden hergestellt werden, siehe z.B. J. Org. Chem. 23 (1958) p. 1595.

Die N-Sulfenyl-N,N′-diacylhydrazide der Formel I können auch durch Behandeln mit Persäuren, wie Peressigsäure, Perbenzoesäure, 3-Chlorperbenzoesäure, Perameisensäure, oder mit Wasserstoffperoxid allein, oder beispielsweise in Lösung mit Aceton oder Methanol, zu den entsprechenden N-Sulfinyl-N,N′-diacylhydraziden der Formel I oxidiert werden, entsprechend der Gleichung:

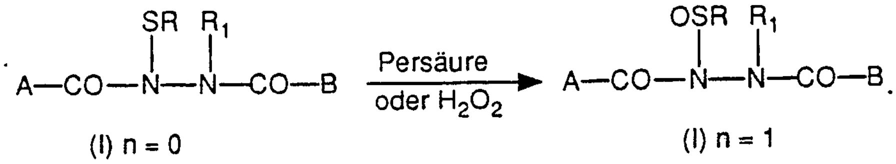


Als Lösungsmittel für diese Oxidation kommen, falls überhaupt nötig, Aceton, Toluol sowie chlorierte Kohlenwasserstoffe in Frage. Die Oxidation findet bei Temperaturen von -20° C bis +50° C, vorzugsweise von -10° C bis +30° C, statt.

Es wurde überraschenderweise gefunden, dass die erfindungsgemässen Verbindungen der Formeln I (bzw. Ia) bei sehr guter Pflanzenverträglichkeit und äussert geringer Warmblütertoxizität ausgezeichnete Wirksamkeit als Schädlingsbekämpfungsmittel aufweisen, insbesondere aufgrund ihrer lang anhaltenden Dauerwirkung. Sie eignen sich vor allem zur Bekämpfung von Pflanzen befallenden Schädlingen.

Es wurde festgestellt, dass die Verbindungen der Formeln I und Ia gute insektizide Eigenschaften besitzen und demzufolge zur Bekämpfung von pflanzenschädigenden bzw. ektoparasitären Insekten, z.B. der Ordnungen Lepidoptera, Coleoptera, Heteroptera, Diptera, Orthoptera und Homoptera, besonders geeignet sind.

Neben ihrer sehr günstigen Wirkung gegenüber Fliegen, wie z.B. Musca domestica, und Mückenlarven eignen sich Verbindungen der Formel I zur Bekämpfung von pflanzenschädigenden Frassinsekten, in Zier- und Nutzpflanzungen, insbesondere in Baumwollkulturen (z.B. Spodoptera littoralis und Heliothis virescens), in Gemüsekulturen (z.B. Leptinotarsa decemlineata und Pieris brassicae) und in Reiskulturen (z.B. Chilo suppressalis).

Die erfindungsgemässen Verbindungen der Formeln I (bzw. Ia) sind auch wirksam gegen pflanzenschädigende Akariden (Spinnmilben: z.B. der Familien Tetranychidae, Tarsonemidae, Eriophyidae, Tyroglyphidae und Glycyphagidae). Einige der erfindungsgemässen Verbindungen zeichnen sich durch gute akarizid-ovizide Aktivität und Blattpenetrationswirkung aus. Die erfindungsgemässen Verbindungen eignen sich vor allem zur Bekämpfung der folgenden, Obst- und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsonemus pallidus, Phyllocoptes vitis und Phyllocoptruta oleivora.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formeln I und Ia werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formeln I und Ia, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden erfindungsgemässen Wirkstoffes oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss-oder Tallöl gewonnen werden können. Ferner sind als Tenside auch die Fettsäure-methyltaurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäure gruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die erfindungsgemäss verwendeten insektiziden Zubereitungen enthalten in der Regel 0,1 bis 99 %, insbesondere 0,1 bis 95 % eines Wirkstoffes der Formel I, gegebenenfalls in Kombination mit anderen Insektiziden oder Akariziden, 1 bis 99,9 % eines festen oder flüssigen Zusatzstoffes und 0 bis 25 %, insbesondere 0,1 bis 25 %, eines Tensides, wobei sich die %-Angaben auf das Gewicht beziehen. Während als Handelsware eher konzentrierte Mittel bevorzugt werden, verwendet der Endverbraucher in der Regel verdünnte Mittel bzw. Zubereitungen, die wesentlich geringere Wirkstoffkonzentrationen, wie z.B. 0,1 bis 1000 ppm, aufweisen. Gewöhnlich beträgt die eingesetzte Aufwandmenge des erfindungsgemäss zu

verwendenden Wirkstoffes - insbesondere für landwirtschaftliche Kulturflächen - 0,025 bis 1,0 kg/ha, vorzugsweise 0,1 bis 0,5 kg/ha, beispielsweise 0,1 bis 0,25 kg/ha.

Die erwähnten Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Im folgenden experimentellen Teil sind die unkorrigierten Schmelzpunkte in °C angegeben.

Beispiel 1: Herstellung von N,N′-Dibenzoyl-N-[(N″-methyl-N″-n-butoxycarbonyl)aminosulfenyl]-N′-tert.butylhydrazid

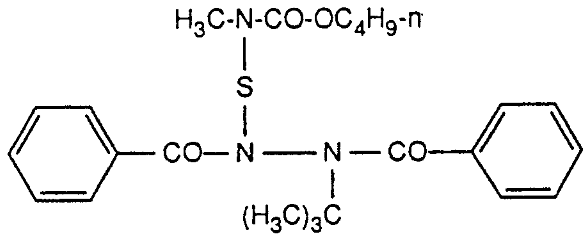

Zu einer Mischung von 8,9 g N,N′-Dibenzoyl-N′-tert.butylhydrazin in 100 ml trockenem Tetrahydrofuran gibt man unter Rühren portionenweise 1,31 g einer 55 % Suspension von Natriumhydrid (NaH) in Mineralöl. Nach dem Abklingen der Wasserstoffentwicklung kühlt man das Reaktionsgemisch auf 0-10°C ab und tropft 5,9 g N-Methyl-N-n-butoxycarbonyl-N-sulfenylchlorid dazu. Das Reaktionsgemisch wird anschliessend weitere 5 Stunden bei Raumtemperatur gerührt, dann filtriert und das Filtrat eingedampft. Der Rückstand wird in Aether aufgenommen, die Lösung filtriert und eingedampft. Das ver bleibende Oel wird über eine Chromatographiesäule von 50cm Länge und 5 cm Durchmesser, welche mit Kieselgel 60 (Merck) gefüllt ist, gereinigt. Als Elutionsmittel wird Hexan/Methylenchlorid (1:1) verwendet; die Fraktionen betragen 200 ml. Die Titelverbindung befindet sich in den Fraktionen 12-20 und wird nach Verdampfen des Lösungsmittels und Verreiben des Rückstandes in etwas Hexan als farblose Kristalle vom Schmelzpunkt 94-95°C erhalten (Verbindung Nr. 1.01).

In analoger Weise wie vorstehend angegeben werden die in der folgenden Tabelle aufgeführten Verbindungen der Formel I hergestellt.

Tabelle 1:

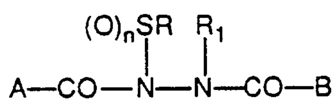

(I)

| No. | A | B | n | R | $R_1$ | phys. Daten |
|---|---|---|---|---|---|---|
| 1.01 | Phenyl | Phenyl | 0 | $-N(CH_3)COOC_4H_9$-n | $-C(CH_3)_3$ | Smp. 94-95°C |
| 1.02 | Phenyl | Phenyl | 0 | $-SC(CH_3)_2CN$ | $-C(CH_3)_3$ | viskoses Oel |
| 1.03 | 4-Cyanophenyl | Phenyl | 0 | $-N(CH_3)COOC_4H_9$-n | $-C(CH_3)_3$ | |
| 1.04 | 4-Trifluormethylphenyl | 4-Trifluormethylphenyl | 0 | $-N(CH_3)COOC_4H_9$-n | $-C(CH_3)_3$ | |
| 1.05 | o-Tolyl | o-Tolyl | 0 | $-N(CH_3)COOC_4H_9$-n | $-C(CH_3)_3$ | |
| 1.06 | 4-Nitrophenyl | 4-Nitrophenyl | 0 | $-N(CH_3)COOC_4H_9$-n | $-C(CH_3)_3$ | |
| 1.07 | 3,4-Dichlorophenyl | Phenyl | 0 | $-N(CH_3)COOC_4H_9$-n | $-C(CH_3)_3$ | |
| 1.08 | Phenoxyphenyl | Phenyl | 0 | $-N(CH_3)COOC_4H_9$-n | $-C(CH_3)_3$ | |
| 1.09 | 2,4-Dichlorophenyl | Phenyl | 0 | $-N(CH_3)COOC_4H_9$-n | $-C(CH_3)_3$ | |
| 1.10 | 3,4-Methylendioxyphenyl | Phenyl | 0 | $-N(CH_3)COOC_4H_9$-n | $-C(CH_3)_3$ | |
| 1.11 | 2-Methoxycarbonylphenyl | Phenyl | 0 | $-N(CH_3)COOC_4H_9$-n | $-C(CH_3)_3$ | |
| 1.12 | 4-Methoxyphenyl | 4-Methoxyphenyl | 0 | $-N(CH_3)COOC_4H_9$-n | $-C(CH_3)_3$ | |
| 1.13 | 4(3,3,3,2,1,1-Hexafluoro-propyl)phenyl | Phenyl | 0 | $-N(CH_3)COOC_4H_9$-n | $-C(CH_3)_3$ | |
| 1.14 | 4-Dimethylaminophenyl | Phenyl | 0 | $-N(CH_3)COOC_4H_9$-n | $-C(CH_3)_3$ | |
| 1.15 | 4-Methylsulfonylphenyl | 4-Methylsulfonylphenyl | 0 | $-N(CH_3)COOC_4H_9$-n | $-C(CH_3)_3$ | |
| 1.16 | 2-Chloro-4-nitrophenyl | 2-Chloro-4-nitrophenyl | 0 | $-N(CH_3)COOC_4H_9$-n | $-C(CH_3)_3$ | |
| 1.17 | Phenyl | Phenyl | 0 | $-N(CH_3)COOC_4H_9$-n | $-C(CN)CH_2CN$ | |
| 1.18 | Phenyl | β-Naphthyl | 0 | $-N(CH_3)COOC_4H_9$-n | $-C(CH_3)_3$ | |
| 1.19 | Phenyl | Phenyl | 0 | Phenyl | $-C(CH_3)_3$ | Smp. 132-134°C |
| 1.20 | Phenyl | Phenyl | 0 | $-N(C_4H_9\text{-n})_2$ | $-C(CH_3)_3$ | $n_D^{40} = 1,5372$ |
| 1.21 | Phenyl | Phenyl | 0 | $-N(CH_3)CON(CH_3)_2$ | $-C(CH_3)_3$ | |
| 1.22 | Phenyl | Phenyl | 0 | $SO_2$Phenyl | $-C(CH_3)_3$ | |
| 1.23 | Phenyl | Phenyl | 0 | $-N(CH_3)COOC_4H_9$-n | -Cyclohexyl | |
| 1.24 | 4-Chlorophenyl | Phenyl | 0 | $-N(CH_3)COOC_4H_9$-n | $-C(CH_3)_3$ | |
| 1.25 | Phenyl | 4-Chlorophenyl | 0 | $-N(CH_3)COOC_4H_9$-n | $-C(CH_3)_3$ | |
| 1.26 | p-Tolyl | p-Tolyl | 0 | $-N(CH_3)COOC_4H_9$-n | $-C(CH_3)_3$ | |
| 1.27 | α-Naphthyl | Phenyl | 0 | $-N(CH_3)COOC_4H_9$-n | $-C(CH_3)_3$ | |
| 1.28 | 2,6-Difluorphenyl | Phenyl | 0 | $-N(CH_3)COOC_4H_9$-n | $-C(CH_3)_3$ | |
| 1.29 | Phenyl | 2,4-Difluorphenyl | 0 | $-N(CH_3)COOC_4H_9$-n | $-C(CH_3)_3$ | |

Tabelle 1 (Fortsetzung)

| No. | A | B | n | R | $R_1$ | phys. Daten |
|---|---|---|---|---|---|---|
| 1.30 | 4-Methoxyphenyl | 4-Methoxyphenyl | 1 | $-N(CH_3)COOC_4H_9$-n | $-C(CH_3)_3$ | |
| 1.31 | Phenyl | β-Naphthyl | 1 | $-N(CH_3)COOC_4H_9$-n | $-C(CH_3)_3$ | |
| 1.32 | α-Naphthyl | Phenyl | 1 | $-N(CH_3)COOC_4H_9$-n | $-C(CH_3)_3$ | |
| 1.33 | Phenyl | Phenyl | 0 | 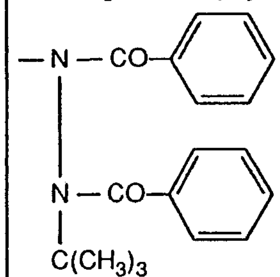 | $-C(CH_3)_3$ | Smp. 138°C |
| 1.34 | 4-Cl-Phenyl | Phenyl | 0 | $-N[-C(CH_3)_3]_2$ | $-C(CH_3)_3$ | $n^{40}_D$ 1,5382 |
| 1.35 | p-Tolyl | Phenyl | 0 | $-N(CH_3)-COOC_4H_9$-t | $-C(CH_3)_3$ | Smp. 100-101°C |
| 1.36 | 4-Cl-Phenyl | Phenyl | 0 | Phenyl | $-C(CH_3)_3$ | Smp. 145-147°C |
| 1.37 | Phenyl | p-Tolyl | 0 | Phenyl | $-C(CH_3)_3$ | Smp. 124-125°C |
| 1.38 | Phenyl | Phenyl | 0 | 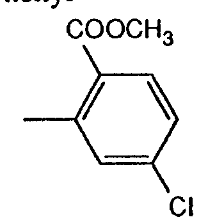 | $-C(CH_3)_3$ | Smp. 153-154°C |
| 1.39 | Phenyl | Phenyl | 0 | 2-Nitrophenyl | $-C(CH_3)_3$ | Smp. 117°C |
| 1.40 | 4-F-Phenyl | Phenyl | 0 | $-N(CH_3)-COOC_4H_9$-t | $-C(CH_3)_3$ | Smp. 88-89°C |
| 1.41 | 3,4-Dichlorphenyl | Phenyl | 0 | $-N(CH_3)-COOC_4H_9$-t | $-C(CH_3)_3$ | Smp. 145-146°C |
| 1.42 | Phenyl | Phenyl | 0 | 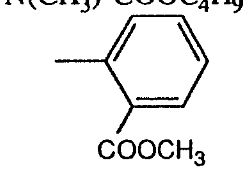 | $-C(CH_3)_3$ | Harz |
| 1.43 | Phenyl | Phenyl | 0 | $-N(C_4H_9-n)\cdot COOCH_3$ | $-C(CH_3)_3$ | Smp. 118-121°C |

Beispiel 2: Wirkung gegen Tetranychus urticae (OP-sensible) und Tetranychus cinnabarinus (OP-tolerant).

Die Primärblätter von Phaseolus vulgaris-Pflanzen wurden 12 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt (Mischpopulation). Die Toleranz bezieht sich auf die Verträglichkeit gegenüber Diazinon.

Die so behandelten infestierten Pflanzen werden mit einer Versuchslösung in Emulsionsform enthaltend 400 ppm der zu prüfenden Verbindung bis zur Tropfnässe besprüht.

Nach 24 Stunden und wiederum nach 6 (T.urticae) bzw. 7 Tagen (T.cinnabarinus) werden Imagines und Larven (alle beweglichen Stadien) sowie abgelegte Eier unter dem Binokular auf lebende und tote Individuen ausgewertet.

Man verwendet pro Testspezies eine Pflanze. Während des Versuchsverlaufs stehen die Pflanzen in Gewächshauskabinen bei 25° C mit etwa 50-60 % rel. Luftfeuchtigkeit.

Die geprüften Verbindungen der Tabelle 1 zeigen in diesem Versuch gute Wirkung gegen Tetranychus urticae und Tetranychus cinnabarinus.

Beispiel 3: Ovizide Wirkung auf Tetranychus urticae (OP-resistent)

Eingetopfte Phaseolus vulgaris-Pflanzen im Primärblatt-Stadium werden jeweils zweimal mit 30 Weibchen von Tetranychus urticae besiedelt. Nach 24-stündiger Eiablage werden die Weibchen mit einer Saugpumpe (Wasserstrahlpumpe) von den Pflanzen entfernt, so dass auf diesen nur noch die abgelegten Milbeneier verbleiben.

Die mit den Milbeneiern infestierten Pflanzen werden dann mit einer wässrigen Emulsion enthaltend 400 ppm der zu prüfenden Verbindung bis zur Tropfnässe besprüht und 5 Tage bei 25° C und etwa 50 % relativer Luftfeuchtigkeit gehalten. Nach dieser Zeit wird die prozentuale Mortalität der Eier und geschlüpften Larven durch Auszählen bestimmt.

Die geprüften Verbindungen der Tabelle 1 zeigen gute Wirkung in obigem Test.

Beispiel 4: Wirkung gegen Musca domestica

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Die geprüften Verbindungen der Tabelle 1 zeigen gute Wirkung im obigen Test.

Beispiel 5: Insektizide Frassgift-Wirkung

Baumwollpflanzen (ca. 20 cm hoch) werden mit wässrigen Wirkstoffemulsionen (erhalten aus einem 10%igen emulgierbaren Konzentrat) besprüht, wobei die Wirkstoffemulsionen 400 ppm der jeweils zu prüfenden Verbindung enthalten.

Nach dem Antrocknen des Belages werden die Baumwollpflanzen mit Spodoptera littoralis- und Heliothis virescens-Larven im dritten larvalen Stadium besetzt. Der Versuch wird bei 24° C und 60 % relativer Luftfeuchtigkeit durchgeführt. In Abständen von jeweils 48 und 96 Stunden werden Mortalität sowie Entwicklungs- und Häutungsstörungen der angesetzten Larven bestimmt.

Die geprüften Verbindungen der Tabelle 1 zeigen gute Wirkung im obigen Test.

Beispiel 6: Wirkung geben Lucilia sericata

Zu 9 ml eines Zuchtmediums wird bei 50 °C 1 ml einer 0,5 % Aktivsubstanz enthaltenden wässrigen Zubereitung gegeben. Nun werden ca. 30 frisch geschlüpfte Lucilia sericata-Larven zum Zuchtmedium hinzugefügt. Nach 48 und 96 Stunden wird die insektizide Wirkung durch Ermittlung der Abtötungsrate festgestellt.

Die geprüften Verbindungen der Tabelle 1 zeigen gute Wirkung im obigen Test.

Beispiel 7: Wirkung geben Aedes aegypti

Auf die Oberfläche von 150 ml Wasser, das sich in einem Behälter befindet, wird so viel einer 0,1 %igen acetonischen Lösung des Wirkstoffes pipettiert, dass eine Konzentration von 400 ppm erhalten wird. Nach Verdunsten des Acetons wird der Behälter mit 30 bis 40 2-tägigen Aedes-Larven beschickt. Nach 2 und 7 Tagen wird die Mortalität geprüft.

Die geprüften Verbindungen der Tabelle 1 zeigen gute Wirkung im obigen Test.

Beispiel 8: Formulierungen für Wirkstoffe der Formeln I bzw. Ia) resp. Kombinationen dieser Wirkstoffe mit andern Insektiziden oder Akariziden (% = Gewichtsprozent):

| 1. Spritzpulver | a) | b) | c) |
|---|---|---|---|
| Wirkstoff gemäss Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

| 2. Emulsions-Konzentrat | |
|---|---|
| Wirkstoff gemäss Tabelle 1 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff gemäss Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 4. Extruder-Granulat | |
|---|---|
| Wirkstoff gemäss Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

| 5. Umhüllungs-Granulat | |
|---|---|
| Wirkstoff gemäss Tabelle 1 | 3 % |
| Polyäthylenglykol (MG 200 | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

| 6. Suspensions-Konzentrat | |
|---|---|
| Wirkstoff gemäss Tabelle 1 | 40 % |
| Aethylenglykol | 10 % |
| Nonylphenolpolyäthylenglykoläther (15 Mol AeO | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Ansprüche**

1. N-Sulfenyl- und N-Sulfinyl-N,N′-diacylhydrazide der Formel I

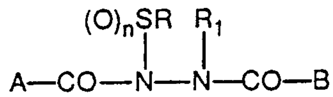 (I)

worin
A und B unabhängig voneinander Phenyl oder Naphthyl, welches unsubstituiert oder ein-oder mehrfach durch Halogen, $C_1$-$C_4$ Alkyl, $C_1$-$C_4$ Alkoxy, $C_1$-$C_4$ Haloalkyl, $C_1$-$C_4$ Haloalkoxy, $C_1$-$C_4$ Alkyl-$S(O)_n$-, $C_1$-$C_4$ Haloalkyl-$S(O)_n$-, Nitro, Cyan, Hydroxyl, Carboxyl oder $C_1$-$C_4$ Alkylcarbonyl oder $C_1$-$C_4$ Alkoxycarbonyl substituiert sind,
n Null oder 1,
R $C_1$-$C_6$ Alkyl, Phenyl oder Naphthyl die unsubstituiert oder wie oben unter A und B angegeben substituiert sein können, oder einen Rest $-N(R_2)_2$, $-N(R_2)COR_2$, $-N(R_2)COOR_2$, $-N(R_2)SO_2R_2$ oder $-(S)_n$-N(COA)N($R_1$)-COB,
$R_1$ $C_1$-$C_6$ Alkyl, $C_1$-$C_6$ Haloalkyl, $C_3$-$C_6$ Alkenyl, $C_3$-$C_6$ Haloalkenyl, $C_3$-$C_6$ Alkynyl, $C_3$-$C_7$ Cycloalkyl, Phenyl oder Naphthyl, welche unsubstituiert oder wie oben unter A und B angegeben substituiert sind
und
$R_2$ $C_1$-$C_6$ Alkyl, $C_1$-$C_6$ Haloalkyl, $C_2$-$C_6$ Alkoxyalkyl, $C_3$-$C_6$ Alkenyl, Phenyl oder Naphthyl, welche unsubstituiert oder wie oben unter A und B angegeben substituiert sind,
bedeuten.

2. Verbindungen gemäss Anspruch 1 der Formel Ia

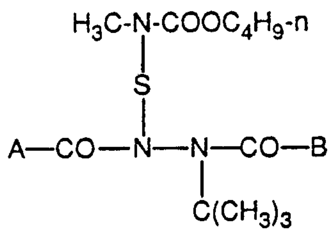 (Ia),

worin A und B unabhängig voneinander einen Phenyl- oder Naphthylrest, welcher, wie in Anspruch 1 unter A und B angegeben, unsubstituiert oder substituiert ist, bedeuten.

3. Verbindungen gemäss Anspruch 1, dadurch gekennzeichnet, dass n für Null steht.

4. Verbindungen gemäss einem der Ansprüche 1 bis 3, dadurch gekennzeichnet, dass A, B, R, $R_1$ und $R_2$ unabhängig voneinander Phenyl, Naphthyl oder einfach oder zweifach, wie in Anspruch 1 angegeben, substituiertes Phenyl oder Naphthyl bedeuten.

5. Verbindungen gemäss Anspruch 4, dadurch gekennzeichnet, dass A und B unabhängig voneinander Phenyl oder mit 1 bis 2 Halogenatomen substituiertes Phenyl bedeuten.

6. Verbindungen gemäss einem der Ansprüche 1,3,4 oder 5, dadurch gekennzeichnet, dass R $C_1$-$C_4$-Alkyl oder einen Rest $N(R_2)_2$ oder $N(R_2)COOR_2$ bedeutet, wobei $R_2$ für $C_1$-$C_4$-Alkyl steht.

7. Verbindungen gemäss einem der Ansprüche 1 bis 6, dadurch gekennzeichnet, dass $R_1$ $-C(CH_3)_3$ bedeutet.

8. N,N′-Dibenzoyl-N-[(N″-methyle-N″-n-butoxycarbonyl)-aminosulfenyl]-N′-tert.butylhydrazid gemäss Anspruch 1.

9. N,N′-Dibenzoyl-N-(2″-cyano-prop-2″-yl-dithio)-N′-tert.butyl-hydrazid gemäss Anspruch 1.

10. Verfahren zur Herstellung der N-Sulfenyl- und N-Sulfinyl-N,N′-diacylhydrazide der Formel I gemäss Anspruch 1, dadurch gekennzeichnet, dass man ein N,N′-Diacylhydrazid der Formel II

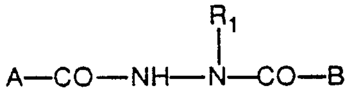 (II),

worin A, B und $R_1$ die im Anspruch 1 angegebenen Bedeutungen haben, in Gegenwart einer Base mit einem Halomerkaptan resp. einer Halosulfinylverbindung der Formel III
$R-[(O)_nS]_m-Hal$ (III),
worin R die im Anspruch 1 angegebene Bedeutung hat, Hal ein Halogenatom, vorzugsweise Chlor oder Brom, n Null oder 1, m 1 oder 2 bedeuten, umsetzt.

11. Verfahren zur Herstellung der N-Sulfenyl- und N-Sulfinyl-N,N′-diacylhydrazide der Formel I gemäss Anspruch 1, worin A und B die gleiche Bedeutung haben, dadurch gekennzeichnet, dass man ein Hydrazin der Formel IV

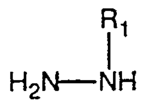

(IV),

worin $R_1$ die im Anspruch 1 angegebene Bedeutung hat, in Gegenwart einer Base, mit einem Halomerkaptan oder einer Halosulfinyl-Verbindung der Formel III

$R\text{-}[(O)_nS]_{\overline{m}}\text{-}Hal$ (III),

unter Bildung einer Verbindung der Formel V

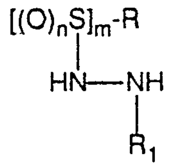

(V),

worin m, n, R und $R_1$ die im Anspruch 1 angegebenen Bedeutungen haben kondensiert und die erhaltene Verbindung der Formel V dann in Gegenwart einer Base mit der zweifach molaren Menge eines Arylcarbonylhalides der Formel VI

A-CO-Hal (VI),

worin A die im Anspruch 1 angegebene Bedeutung hat und Hal ein Halogenatom, vorzugsweise Chlor oder Brom bedeutet, umsetzt.

12. Verfahren zur Herstellung der N-Sulfenyl- oder N-Sulfinyl-N,N′-diacylhydrazide der Formel I gemäss Anspruch 1, dadurch gekennzeichnet, dass man ein Acylhydrazid der Formel VII

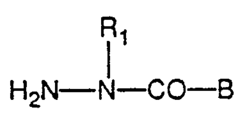

(VII),

worin B und $R_1$ die im Anspruch 1 angegebenen Bedeutungen haben in Gegenwart einer Base zuerst mit einem Halomerkaptan oder einer Halosulfinylverbindung der Formel III kondensiert

$R\text{-}[(O)_nS]_{\overline{m}}\text{-}Hal$ (III),

worin Hal, m, n und R die im Anspruch 1 angegebenen Bedeutungen haben, und das erhaltene N-Sulfenyl- oder N-Sulfinyl-N′-acyl-hydrazid der Formel VIII

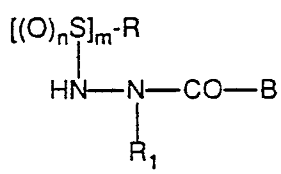

(VIII),

worin B, m, n, R und $R_1$ die im Anspruch 1 angegebenen Bedeutungen haben, dann in Gegenwart der einer Base mit einem Arylcarbonylhalid der Formel VI A-CO-Hal (VI), worin A die unter der Formel I gegebene Bedeutung hat und Hal für ein Halogenatom, vorzugsweise Chlor oder Brom, steht, umsetzt.

13. Verfahren zur Herstellung von N-Sulfinyl-N,N′-diacyl-hydraziden der Formel I gemäss Anspruch 1, dadurch gekennzeichnet, dass man ein N-Sulfenyl-N,N′-diacylhydrazid der Formel I durch Behandeln mit Persäuren oder Wasserstoffperoxid oxidiert.

14. Mittel zum Bekämpfen von Schädlingen, welches neben inerten Bestandteilen als aktive Komponente eine insektizid bzw. akarizid wirksame Menge eines N-Sulfenyl- oder N-Sulfinyl-N,N′-diacylhydrazides der Formel I gemäss Anspruch 1 enthält.

15. Verwendung einer Verbindung gemäss Anspruch 1 zur Bekämpfung von Insekten und Vertretern

der Ordnung Akarina an Pflanzen.

16. Verwendung gemäss Anspruch 15 zur Bekämpfung von pflanzenschädigenden Milben.

17. Verwendung gemäss Anspruch 15 zur Bekämpfung von pflanzenschädigenden Insekten.

18. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung gemäss Anspruch 1 oder mit einem Mittel enthaltend neben Zusatz- und Trägerstoffen eine pestizid wirksame Menge dieser Verbindung, in Kontakt bringt oder behandelt.

19. Verfahren gemäss Anspruch 18 zur Bekämpfung pflanzenschädigender Milben.

20. Verfahren gemäss Anspruch 18 zur Bekämpfung pflanzenschädigender Insekten.

European Patent Office

# EUROPEAN SEARCH REPORT

Application number

EP 90810306.2

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| A | EP - A1 - 0 105 525 (MAX-PLANCK-GESELLSCHAFT) * Anspruch 1 * -- | 1 | C 07 C 381/08<br>C 07 C 313/24<br>C 07 C 381/00<br>A 01 N 41/00 |
| A | DE - A - 2 314 380 (IMPERIAL CHEMICAL INDUSTRIES) * Ansprüche 1,6 * -- | 1,10 | |
| A | EP - A1 - 0 028 584 (CIBA-GEIGY) * Ansprüche 1,6-9 * ---- | 1,10, 14-20 | |

| TECHNICAL FIELDS SEARCHED (Int Cl 5) |
|---|
| C 07 C 381/00<br>C 07 C 313/00<br>C 07 C 243/00 |

The present search report has been drawn up for all claims

| Place of search | Date of completion of the search | Examiner |
|---|---|---|
| WIEN | 18-07-1990 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document